# EUROPEAN PATENT APPLICATION

(11) **EP 4 303 569 A1**
(43) Date of publication of application: **10.01.2024**
(21) Application number: 22791742.4
(22) Date of filing: 19.04.2022
(51) Int. Cl.: G01N 21/64, C12Q 1/04, G01N 33/02

(54) **DETERMINATION DEVICE**

(30) Priority: 20.04.2021 JP 2021071354
(71) Applicant: Daikin Industries, Ltd., Osaka-shi, Osaka 530-0001 (JP); University of Miyazaki, Miyazaki-shi Miyazaki 889-2192 (JP)
(72) Inventor: KOBAYASHI Taichi, Miyazaki-shi, Miyazaki 889-2192 (JP); SAKAI Kentaro, Miyazaki-shi, Miyazaki 889-2192 (JP); HIYOSHI Kenji, Miyazaki-shi, Miyazaki 889-2192 (JP); MATSUMOTO Tomoko, Miyazaki-shi, Miyazaki 889-2192 (JP); GEJIMA Yoshinori, Miyazaki-shi, Miyazaki 889-2192 (JP); NISHIMOTO Motomi, Osaka-shi, Osaka 530-0001 (JP); SATO Kiichiro, Osaka-shi, Osaka 530-0001 (JP); NAKANO Masataka, Osaka-shi, Osaka 530-0001 (JP); NAMIKAWA Takashi, Osaka-shi, Osaka 530-0001 (JP); MAEDA Shotaro, Osaka-shi, Osaka 530-0001 (JP); KAWAI Shogo, Osaka-shi, Osaka 530-0001 (JP); YANAGI Yuri, Osaka-shi, Osaka 530-0001 (JP)
(74) Representative: Goddar, Heinz J.
(86) International application number: PCT/JP2022/018225
(87) International publication number: WO 2022/224971

(57) **Abstract**

A determination device includes: an irradiator (10) configured to irradiate an object (A) including a food or a plant with a light; an extractor (20) configured to extract a predetermined fluorescence emission having a predetermined wavelength out of fluorescence emissions generated from a surface of the object (A) irradiated with the light; an imager (30) configured to capture a fluorescence image indicating the predetermined fluorescence emission; and a determiner (60) configured to determine a state of the object (A) based on an index indicating a fluorescence intensity of the fluorescence image.

## Description

### TECHNICAL FIELD

The present disclosure relates to a determination device.

### BACKGROUND ART

A device for determining the degree of freshness/maturity described in Patent Document 1 includes a container, a light source, an optical sensor, and a control unit. The container stores fresh food. The light source irradiates the fresh food placed in the container with visible light. The optical sensor detects the light intensity of a predetermined wavelength region of light reflected from the fresh food irradiated with the visible light. The control unit determines the degree of freshness or maturity of the fresh food based on the light intensity of the predetermined wavelength region detected by the optical sensor.

### CITATION LIST

### PATENT DOCUMENT

Patent Document 1: Japanese Unexamined Patent Publication No. 2018-096712

### SUMMARY OF THE INVENTION

### TECHNICAL PROBLEM

The device for determining the degree of freshness/maturity described in Patent Document 1 determines only the states of points of the fresh food irradiated with visible light. Thus, if the state of whole fresh food as an object is determined, the accuracy of this determination may decrease.

It is an object of the present disclosure to reduce a decrease in accuracy of determination of the state of an object.

### SOLUTION TO THE PROBLEM

A first aspect of the present disclosure is directed to a determination device. The determination device includes: an irradiator (10) configured to irradiate an object (A) including a food or a plant with a light; an extractor (20) configured to extract a predetermined fluorescence emission having a predetermined wavelength out of fluorescence emissions generated from a surface of the object (A) irradiated with the light; an imager (30) configured to capture a fluorescence image indicating the predetermined fluorescence emission; and a determiner (60) configured to determine a state of the object (A) based on an index indicating a fluorescence intensity of the fluorescence image.

The first aspect can reduce a decrease in accuracy of determination of the state of the object (A).

A second aspect of the present disclosure is an embodiment of the first aspect. In the second aspect, the determiner (60) determines the state of the object (A) based on the fluorescence image including a plurality of fluorescence images, and the predetermined fluorescence emission indicated by the fluorescence image has a different wavelength for each of the fluorescence images.

According to the second aspect, the determiner (60) uses the plurality of fluorescence images to determine the state of the object (A), thereby further improving the determination accuracy.

A third aspect of the present disclosure is an embodiment of the first or second aspect. In the third aspect, the determination device includes a scanning mechanism (70) configured to change relative positions of the object (A) and the imager (30) relative to each other.

According to the third aspect, the fluorescence image of the entire object (A) can be acquired.

A fourth aspect of the present disclosure is an embodiment of the first to third aspects. In the fourth aspect, the predetermined wavelength is 550 nm or more and 750 nm or less.

According to the fourth aspect, the extractor (20) can extract the predetermined fluorescence emission having a wavelength of 550 nm or more and 750 nm or less.

A fifth aspect of the present disclosure is an embodiment of any one of the first to fourth aspects. In the fifth aspect, the irradiator (10) emits ultraviolet rays.

According to the fifth aspect, ultraviolet rays are emitted from the irradiator (10), and thus a fluorescence emission can be generated from the object (A).

A sixth aspect of the present disclosure is an embodiment of the fifth aspect. In the sixth aspect, the ultraviolet rays have a wavelength of 200 nm or more and 400 nm or less.

According to the sixth aspect, the irradiator (10) can emit ultraviolet rays having a wavelength of 200 nm or more and 400 nm or less.

A seventh aspect of the present disclosure is an embodiment of any one of the first to sixth aspects. In the seventh aspect, the imager (30) captures the fluoroscopic image over time, and the determiner (60) determines whether or not there is a sign of deterioration in quality of the object (A) based on the index of the fluorescence image captured over time.

According to the seventh aspect, by checking the index indicating the fluorescence intensity of the fluorescence image acquired over time, it is possible to check a temporal change in the state of the object (A).

An eighth aspect of the present disclosure is an embodiment of any one of the first to sixth aspects. In the eighth aspect, the extractor (20) extracts the predetermined fluorescence emission including a plurality of predetermined fluorescence emissions each having a different wavelength, the imager (30) performs an imaging process of capturing a fluorescence image of each of the predetermined fluorescence emissions over time, and the determiner (60) determines whether or not there is a sign of deterioration in quality of the object (A) based on the index of each of the fluorescence images acquired through the imaging process performed over time.

According to the eighth aspect, by checking the indexes each indicating the fluorescence intensity of the associated fluorescence image acquired through the imaging process performed over time, it is possible to check a temporal change in the state of the object (A).

A ninth aspect of the present disclosure is an embodiment of any one of the first to eighth aspects. In the ninth aspect, the extractor (20) includes a band-pass filter.

According to the ninth aspect, the band-pass filter can extract the predetermined fluorescence emission.

A tenth aspect of the present disclosure is an embodiment of the ninth aspect. In the tenth aspect, the determination device further includes the band-pass filter including a plurality of band-pass filters, and a switching part (80) configured to switch one of the plurality of band-pass filters that is used to extract the predetermined fluorescence emission; and each of the band-pass filters extracts the predetermined fluorescence emission having a different wavelength.

According to the tenth aspect, the switching part switching the plurality of band-pass filters enables easy acquisition of the plurality of fluorescence images of the predetermined fluorescence emissions having different wavelengths.

An eleventh aspect of the present disclosure is an embodiment of any one of the first to tenth aspects. In the eleventh aspect, the determination device further includes the extractor (20) including a plurality of extractors (20), and the imager (30) including a plurality of the imagers (30); and each of the extractors (20) extracts the predetermined fluorescence emission having a different wavelength; and the plurality of extractors (20) are associated with the plurality of imagers (30).

According to the eleventh aspect, the plurality of extractors (20) and the plurality of imagers (30) are used, such that the plurality of fluorescence images having different wavelengths can be acquired all at once. Thus, the process of determining the state of the object (A) can be performed efficiently.

A twelfth aspect of the present disclosure is an embodiment of any one of the first to eleventh aspects. In the twelfth aspect, the irradiator (10) emits the light including a plurality of lights having different excitation wavelengths, and the determiner (60) determines the state of the object (A) based on the index including a plurality of indexes associated with the plurality of lights.

According to the twelfth aspect, a fluorescence fingerprint including the plurality of indexes is used, such that the state of the object (A) can be determined.

A thirteenth aspect of the present disclosure is an embodiment of the twelfth aspect. In the thirteenth aspect, the determiner (60) identifies a type of a live bacterium or a microorganism that is parasitic on the object (A) based on the plurality of indexes.

In the thirteenth aspect, the fluorescence fingerprint including the plurality of indexes is used, such that the type of the live bacterium or the microorganism involved in degradation in the object (A) can be identified.

### BRIEF DESCRIPTION OF THE DRAWINGS

FIG. 1 is a block diagram illustrating a configuration of a determination device according to a first embodiment of the present invention.
FIG. 2 illustrates fluorescence images of fluorescence emissions having wavelengths of 420 nm to 750 nm.
FIG. 3 illustrates a switching part.
FIG. 4 illustrates a variation of the determination device (1) according to the first embodiment.
FIG. 5 shows a fluorescence fingerprint.
FIG. 6 illustrates an example of data indicating association among an image of an object, a fluorescence color image of the object, and fluorescence images of the object for each of a plurality of observation days.
FIG. 7 illustrates an example of data indicating association among an image of an object, a fluorescence color image of the object, and fluorescence images of the object for each of a plurality of observation days.
FIG. 8 illustrates an example of data indicating association among an image of an object, a fluorescence color image of the object, and fluorescence images of the object for each of a plurality of observation days.
FIG. 9 is an example of a graph showing an index indicating the fluorescence intensity on each of a plurality of observation days.
FIG. 10 is an example of a graph showing an index indicating the fluorescence intensity on each of a plurality of observation days.
FIG. 11 is an example of a graph showing an index indicating the fluorescence intensity on each of a plurality of observation days.
FIG. 12 is an example of a graph showing an index indicating the fluorescence intensity on each of a plurality of observation days.
FIG. 13 is an example of a graph showing an index indicating the fluorescence intensity on each of a plurality of observation days.
FIG. 14 is an example of a graph showing an index indicating the fluorescence intensity on each of a plurality of observation days.
FIG. 15 is a flowchart relating to a determination made by the determination device.

### DESCRIPTION OF EMBODIMENTS

Embodiments of the present invention will be described in detail with reference to the drawings. Note that like reference characters denote the same or equivalent components in the drawings, and the detailed description thereof, the description of advantages associated therewith, and other descriptions will not be repeated.

### -First Embodiment-

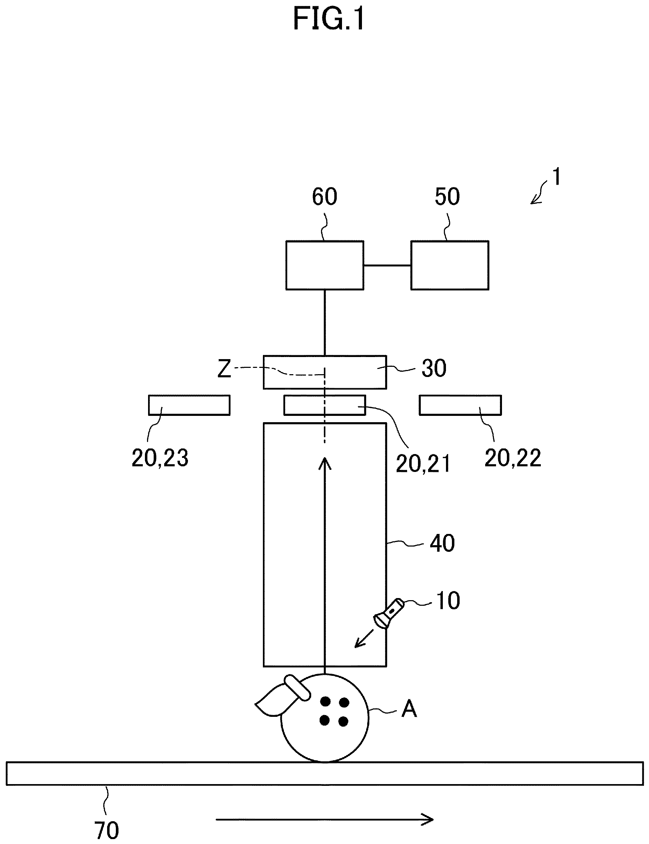

A determination device (1) according to a first embodiment of the present invention will be described with reference to FIG. 1. FIG. 1 is a block diagram illustrating a configuration of the determination device (1) according to the first embodiment of the present invention.

### -General Configuration-

As illustrated in FIG. 1, the determination device (1) includes an irradiator (10), extractors (20), an imager (30), a guide (40), a storage (50), and a determiner (60).

The irradiator (10) irradiates an object (A) with light. The object (A) of the first embodiment is a substance that fluoresces when irradiated with light. The object (A) is, for example, a food or a plant.

In the first embodiment, the irradiator (10) irradiates the object (A) with light. In the first embodiment, the irradiator (10) emits ultraviolet rays. The ultraviolet rays emitted by the irradiator (10) have a wavelength of 200 nm or more and 400 nm or less, for example. Examples of the irradiator (10) include a light source that emits ultraviolet rays. Examples of the light source include a light source element, such as a light-emitting diode (LED) and a laser diode, an excimer lamp, an ultraviolet lamp, and a mercury lamp. The irradiator (10) irradiates the object (A) with ultraviolet rays, such that fluorescence is emitted from the surface of the object (A).

The extractors (20) extract a fluorescence emission having a predetermined wavelength out of the fluorescence emissions generated from the surface of the object (A). The predetermined wavelength is, for example, 550 nm or more and 750 nm or less. Examples of the extractors (20) include a band-pass filter. The number of the extractors (20) is two or more. In the first embodiment, the extractors (20) include a first extractor (21), a second extractor (22) and a third extractor (23). The first, second, and third extractors (21), (22), and (23) extract fluorescence emissions having wavelengths of 550 nm, 750 nm, and 400 nm, respectively, out of the fluorescence emissions generated from the surface of the object (A).

One of the extractors (20) to be used faces the object (A). In FIG. 1, the first extractor (21) is used. The first extractor (21) extracts a fluorescence emission having a wavelength of 550 nm out of the fluorescence emissions generated from the surface of the object (A).

The fluorescence emission extracted by the extractor (20) may be hereinafter referred to as the "predetermined fluorescence emission."

The imager (30) captures a fluorescence image showing the predetermined fluorescence emission. Examples of the imager (30) include a camera.

The guide (40) is disposed between the object (A) and the extractor (20). The guide (40) is a tubular member where both ends open near the object (A) and near the extractor (20). The guide (40) guides the fluorescence emissions generated from the object (A) toward the extractor (20). The guide (40) effectively allows the fluorescence emissions to have a sufficiently high directivity toward the extractor (20).

The storage (50) includes a main memory (e.g., a semiconductor memory), such as a flash memory, a read only memory (ROM), and a random access memory (RAM), and may further include an auxiliary memory (e.g., a hard disk drive, a solid state drive (SSD), a secure digital (SD) memory card, or a universal serial bus (USB) flash memory). The storage (50) stores various computer programs executable by the determiner (60).

The determiner (60) includes a processor, such as a central processing unit (CPU) or a microprocessor unit (MPU). The determiner (60) executes a computer program stored in the storage (50) so as to control elements of the determination device (1).

The determiner (60) determines the state of the object (A) based on an index indicating the fluorescence intensity of the fluorescence image captured by the imager (30).

### -Principle of Determining State of Object-

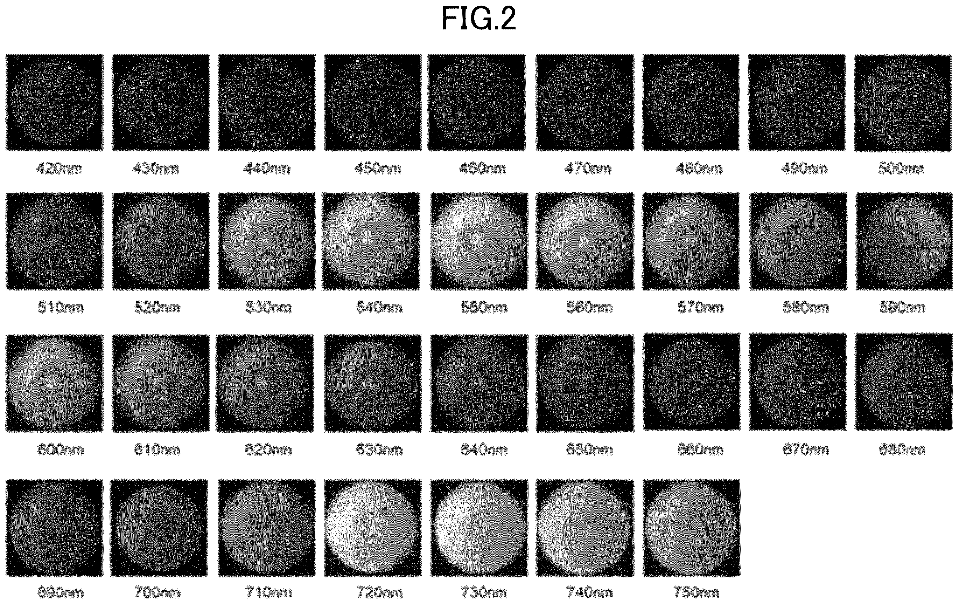

FIG. 2 illustrates fluorescence images of fluorescence emissions having wavelengths of 420 nm to 750 nm. As illustrated in FIG. 2, the fluorescence images of the same object (A) vary depending on the types of the extractors (20) (e.g., depending on which one is used among the extractor (20) that extracts a fluorescence emission having a wavelength of 420 nm and the extractor (20) that extracts a fluorescence emission having a wavelength of 750 nm is used). Even if the extractor (20) of the same type is used (e.g., even if only the extractor (20) that extracts a fluorescence emission having a wavelength of 420 nm is used), the fluorescence images vary depending on the state of the object (A).

In the first embodiment, the state of the object (A) is determined using the correlation among the state of the object (A), the fluorescence images, and the type of the extractor (20).

### -Procedure of Determining State of Object-

As illustrated in FIG. 1, the first extractor (21) is used to extract a fluorescence emission having a wavelength of 550 nm out of the fluorescence emissions generated from the object (A). The imager (30) captures a fluorescence image indicating the fluorescence emission having a wavelength of 550 nm (a 550-nm fluorescence image). The determiner (60) outputs an index λ1 indicating the fluorescence intensity of the 550-nm fluorescence image. The index indicating the fluorescence intensity indicates, for example, an index value of pixel values of a plurality of pixels that constitute the fluorescence image. The index value indicates the mean value, the median value, the mode, the maximum value, or the minimum value. The index indicating the fluorescence intensity may be obtained by converting the index value of the pixel values into an optical value, such as lumen, candela, or lux, using a predetermined transformation, a table, or the like. The index indicating the fluorescence intensity may be an absolute value, or may be a relative value.

The second extractor (22) is used to extract a fluorescence emission having a wavelength of 750 nm out of the fluorescence emissions generated from the object (A). The imager (30) captures a fluorescence image indicating the fluorescence emission having a wavelength of 750 nm (a 750-nm fluorescence image). The determiner (60) outputs an index λ2 indicating the fluorescence intensity of the 750-nm fluorescence image.

The determiner (60) outputs a predicted value X of the chlorophyll content in the object (A) using the index λ1, the index λ2, and the following formula 1. The formula 1 is stored in the storage (50).

[Formula 1] X = 13.35 + 10.74λ2 - 82.62λ1

The formula 1 is obtained, for example, by determining the correlation among the index λ1 indicating the fluorescence intensity of the 550-nm fluorescence image, the index λ2 indicating the fluorescence intensity of the 750-nm fluorescence image, and a measured value of the chlorophyll content through multivariate analysis or the like, and then expressing that correlation in the form of an approximate expression.

The formula 1 is determined according to the type of the object (A). For example, if there is a strong correlation among an index λ3 indicating the fluorescence intensity of a 400-nm fluorescence image, the index λ1 indicating the fluorescence intensity of the 550-nm fluorescence image, and the measured value of the chlorophyll content, the formula 1 is expressed in the form of the approximate expression of the correlation among them. In this case, when the chlorophyll content in the object (A) is output using the formula 1, the third extractor (23) for a wavelength of 400 nm and the first extractor (21) for a wavelength of 550 nm are used.

The determiner (60) compares the predicted value X of the chlorophyll content in the object (A) output using the formula 1 with a predetermined threshold value. If the predicted value X is the predetermined threshold value or more, the determiner (60) determines that the object (A) has a high degree of freshness (a non-defective product). If the predicted value X is less than the predetermined threshold value, the determiner (60) determines that the object (A) has a low degree of freshness (a defective product).

Artificial intelligence (AI) may be used to determine whether the object (A) is a non-defective product or a defective product. In this case, a known machine learning device, such as a neural network, is used to learn features of the fluorescence image observed when the object (A) is a non-defective product and features of the fluorescence image observed when the object (A) is a defective product, in order to determine whether the object (A) shown in a newly input fluorescence image is a non-defective product or a defective product.

### -Advantages of First Embodiment-

As described above, the determiner (60) determines the state of the object (A) based on the indexes each indicating the fluorescence intensity of a fluorescence image. The fluorescence image is used as described above, such that the entire surface of the object (A) that fluoresces in response to light emitted from the irradiator (10) can be captured as a target for determination. Thus, a larger part of the surface of the object (A) can be used for determination than in a typical spot determination process. Then, the determination accuracy can be improved.

### -Second Embodiment-

In the first embodiment, the formula 1 contains two variables: the indexes λ1 and λ2, but may contain one variable. For example, the variable in the formula 1 may include the index λ1 only. In this case, extractors (20) only have to include a first extractor (21). In other words, the extractors (20) may include one extractor. In this case, the formula 1 is obtained by determining the correlation between the index λ1 indicating the fluorescence intensity of a 550-nm fluorescence image and a measured value of the chlorophyll content through multivariate analysis or the like, and then expressing that correlation in the form of an approximate expression.

### -Third Embodiment-

In the first embodiment, the formula 1 contains two variables: the indexes λ1 and λ2, but may contain three or more variables. For example, the variables in the formula 1 may include the indexes λ1 and λ2 and an index λX. The index λX indicates the fluorescence intensity of an X-nm fluorescence image (a fluorescence image indicating a fluorescence emission having a wavelength of X nm). In this case, extractors (20) include a first extractor (21), a second extractor (22), and an X-th extractor. The X-th extractor extracts a fluorescence emission having a wavelength of X nm. In this case, the formula 1 is obtained by determining the correlation among the index λ1 indicating the fluorescence intensity of the 550-nm fluorescence image, the index λ2 indicating the fluorescence intensity of the 750-nm fluorescence image, the index λX indicating the fluorescence intensity of the X-nm fluorescence image, and a measured value of the chlorophyll content through multivariate analysis or the like, and then expressing that correlation in the form of an approximate expression.

### -Fourth Embodiment-

In each of the first to third embodiments, the determiner (60) determines the degree of freshness of the object (A) as a state of the object (A). However, the present invention is not limited to this. For example, the determiner (60) may make determination for a ripened portion of the object (A) such as black spots on a banana, a rotten portion (a portion that has turned black) of the object (A), or the like, based on the pixel values (color shade) of the fluorescence images of the object (A).

### -Fifth Embodiment-

In each of the first to fourth embodiments, when using the fluorescence images to determine the state of the object (A), the determiner (60) uses all of the fluorescence images to determine the state of the entire imaged range of the object (A). However, the present invention is not limited to this. When using the fluorescence images to determine the state of the object (A), the determiner (60) may use part of the fluorescence images to determine the state of a partial region of the imaged range of the object (A) (such as an especially perishable region of the object (A)).

### -Sixth Embodiment-

As illustrated in FIG. 1, the determination device (1) may include a scanning mechanism (70). The scanning mechanism (70) changes relative positions of the object (A) and each of the extractors (20) and the imager (30) relative to each other. Examples of the scanning mechanism (70) include a conveyor that conveys the object (A).

If the object (A) is partially outside the imaged range of the imager (30), and an image of the entire object (A) cannot be captured in a single shot, the conveyor conveys the object (A) while the imager (30) successively captures images of the object (A), such that it is possible to easily capture an image of the entire object (A). The scanning mechanism (70) may move the extractors (20) and the imager (30). In this case, examples of the scanning mechanism (70) include a link mechanism, a slide mechanism, or a robot arm to which the extractors (20) and the imager (30) are attached.

### -Sixth Embodiment-

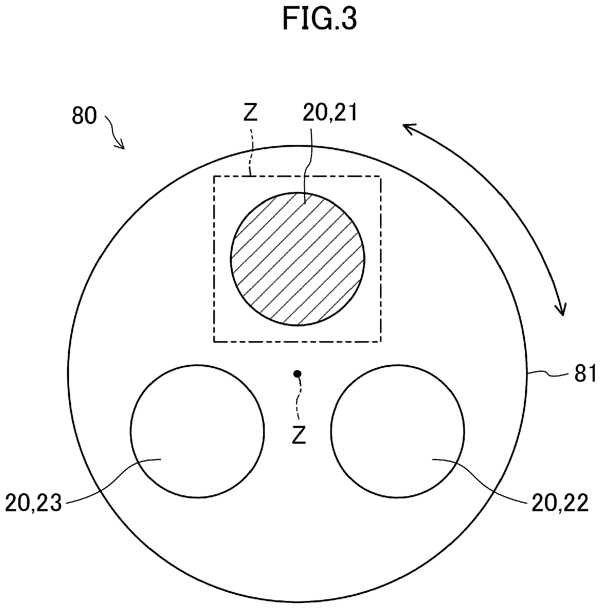

As illustrated in FIG. 3, a determination device (1) may include a switching part (80). The switching part (80) switches an extractor (20) of a plurality of extractors (20) that is used when an imager (30) captures a fluorescence image. The switching part (80) include a support (81), for example. The support (81) is disposed between the imager (30) and a guide (40) (see FIG. 1). The support (81) is supported rotatably about the Z axis. The support (81) is installed such that the plurality of extractors (20) are arranged around the Z axis. The plurality of extractors (20) rotate together with the support (81). The imager (30) captures a fluorescence image by using an extractor (20) of the plurality of extractors (20) that is disposed below the imager (30). The switching part (80) illustrated in FIG. 3 uses the first extractor (21) for the imager (30) to capture a fluorescence image. If the rotational angle of the support (81) is changed, an extractor (20) of the plurality of extractors (20) that is used when the imager (30) captures a fluorescence image is switched on. Accordingly, the extractors (20) can be easily switched.

### -Seventh Embodiment-

A determination device (1) may include a plurality of extractors (20) and a plurality of imagers (30). For example, if a plurality of extractors (20) include first to third extractors (21) to (23) (see FIG. 1), three imagers (30) (first to third imagers) are provided. The first to third extractors (21) to (23) are configured to be associated with the first to third imagers, respectively. An image of a fluorescence emission extracted by the first extractor (21) is captured by the first imager, an image of a fluorescence emission extracted by the second extractor is captured by the second imager, and an image of a fluorescence emission extracted by the third extractor is captured by the third imager. As a result, a plurality of fluorescence images with different wavelengths can be captured in a single shot. Thus, the determiner (60) can efficiently determine the state of the object (A).

### -Eighth Embodiment-

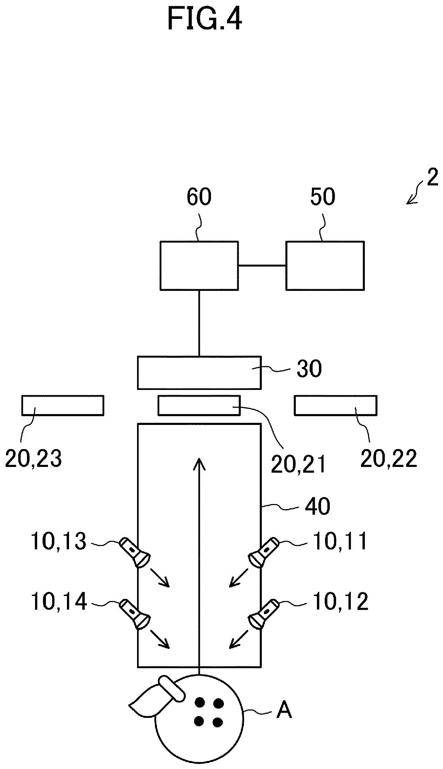

A determination device (2) that is a variation of the determination device (1) will be described with reference to FIG. 4. Differences from the determination device (1) will be mainly described below.

As illustrated in FIG. 4, the determination device (2) includes a plurality of irradiators (10). The plurality of irradiators (10) emit ultraviolet rays each having a different excitation wavelength. In the eighth embodiment, the plurality of irradiators (10) include a first irradiator (11) that emits 325-nm ultraviolet rays, a second irradiator (12) that emits 365-nm ultraviolet rays, a third irradiator (13) that emits 375-nm ultraviolet rays, and a fourth irradiator (14) that emits 385-nm ultraviolet rays.

The determination device (2) may include a plurality of extractors (20) or a single extractor (20).

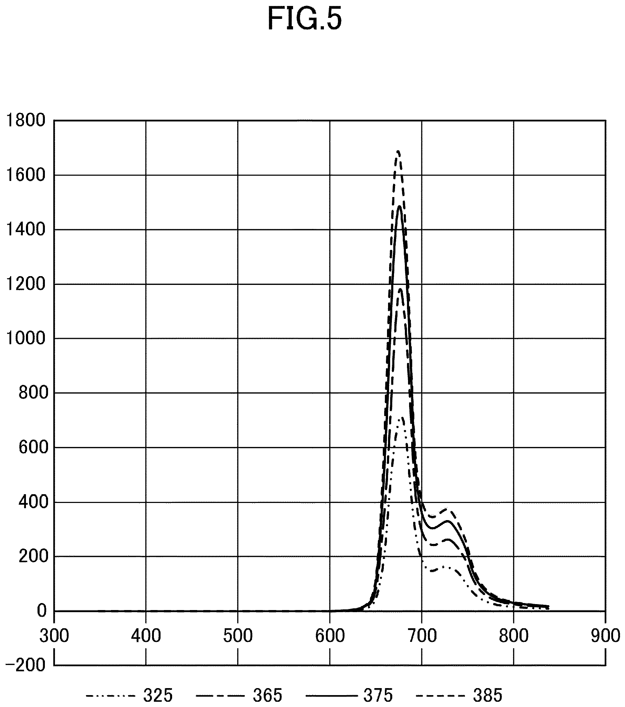

As illustrated in FIG. 5, if the excitation wavelengths of ultraviolet rays emitted to the object (A) from the plurality of irradiators (10) (the first to fourth irradiators (11) to (14)) are changed, the data can be obtained in the form of three-dimensional contours, including the excitation wavelength, the fluorescence wavelength, and the fluorescence intensity. The data in the form of three-dimensional contours may be hereinafter referred to as the "fluorescence fingerprint." The fluorescence fingerprint is obtained for each of pixels of a fluorescence image. FIG. 5 shows a fluorescence fingerprint of one pixel in a fluorescence image of the object (A), where the imager (30) captures the fluorescence image by using each of the first irradiator (11) that emits 325-nm ultraviolet rays, the second irradiator (12) that emits 365-nm ultraviolet rays, the third irradiator (13) that emits 375-nm ultraviolet rays, and the fourth irradiator (14) that emits 385-nm ultraviolet rays. If a plurality of extractors (20) are used as illustrated in FIG. 4 so that a fluorescence image is captured for each of the extractors (20), a fluorescence fingerprint (see FIG. 5) is obtained for each pixel of the fluorescence image and further obtained for each extractor (20). If the plurality of extractors (20) are used as described above, fluorescence fingerprints each obtained for each pixel of the fluorescence image and further obtained for each extractor (20) may be collectively referred to as a "fluorescence fingerprint group."

Note that the number of the extractors (20) to be used may be one. In this case, only one extractor (20) is used. and thus a fluorescence fingerprint is obtained for each pixel of a fluorescence image. If the only one extractor (20) is used as described above, fluorescence fingerprints each obtained for each pixel of the fluoroscopic image may be collectively referred to as a "fluorescence fingerprint group."

A configuration in which a determiner (60) determines the state of the object (A) using the fluorescence fingerprint group will be described.

The storage (50) illustrated in FIG. 4 stores first association information indicating association between a plurality of different fluorescence fingerprint groups and a plurality of different states of the object (A). When the object (A) as a target for determination is irradiated with a plurality of ultraviolet rays having different wavelengths, and the determiner (60) acquires information indicating the fluorescence fingerprint group of the object (A) as a target for determination, the determiner (60) selects, from the first association information, the state of the object (A) associated with the acquired fluorescence fingerprint group. In this case, the determiner (60) may use multivariate analysis, AI analysis, data mining, or the like to select the state of the object (A). The determiner (60) outputs the state of the object (A) selected from the first association information as the state of the object (A) as a target for determination. As a result, the determiner (60) can use the fluorescence fingerprints of the object (A) to determine the state of the object (A).

### -Ninth Embodiment-

In a ninth embodiment, a determination device (2) is used to identify the type of live bacteria or microorganisms that are parasitic on an object (A). In this case, the storage (50) illustrated in FIG. 4 stores second association information indicating association between a plurality of different fluorescence fingerprint groups and a plurality of different types of live bacteria or microorganisms. When the object (A) as a target for determination is irradiated with a plurality of ultraviolet rays having different wavelengths, and a determiner (60) acquires information indicating the fluorescence fingerprint group of the object (A) as a target for determination, the determiner (60) selects, from the second association information, the live bacteria or microorganisms associated with the acquired fluorescence fingerprint group. In this case, the determiner (60) may use multivariate analysis, AI analysis, data mining, or the like to select the live bacteria or microorganisms. The determiner (60) outputs the live bacteria or microorganisms selected from the second association information as the live bacteria or microorganisms that are parasitic on the object (A) as a target for determination. As a result, the determiner (60) can use the fluorescence fingerprints of the object (A) to identify the type of live bacteria or microorganisms involved in degradation in the object (A).

### -Tenth Embodiment-

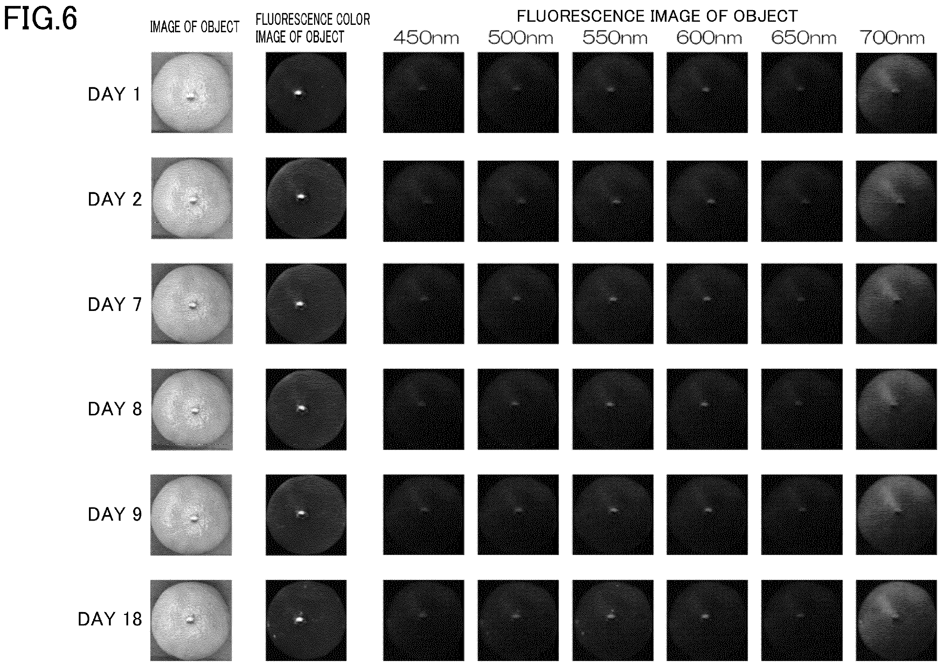

In a tenth embodiment, a determiner (60) of a determination device (1, 2) determines whether or not there is a sign of deterioration in quality of an object (A), which is an example of the state of the object (A). The inventors of this application observed the object (A) over a predetermined period to acquire fluorescence images of the object (A) on each of observation days, thereby acquiring data shown in FIGS. 6 to 14.

### -Principle of Determining Sign of Deterioration in Quality of Object-

FIGS. 6 to 8 illustrate data indicating association among an image of the object (A), a fluorescence color image of the object (A), and fluorescence images of the object (A) for each of a plurality of observation days. To examine the correlation between a temporal change in the object (A) and the fluorescence images, the inventors of this application observed the object (A), captured an image of the object (A) on each of the observation days, used the determination device (1, 2) shown in FIG. 1 or 4 to acquire fluorescence images, and accordingly acquired the data shown in FIGS. 6 to 9. In the tenth embodiment, the object (A) shown in FIGS. 6 to 9 is a citrus fruit, such as Hebesu.

In FIGS. 6 to 8, the day number, such as day 1 or day 2, indicates the day of observation. In FIGS. 6 to 8, the images of the object (A) are images of the object (A) directly captured. The images of the object (A) shown in FIGS. 6 to 9 are actually color images showing the colors of the outer surface of the object (A). In FIGS. 6 to 8, the fluorescence color images of the object (A) are images captured without extracting a predetermined fluorescence emission out of fluorescence emissions generated from the surface of the object (A). The fluorescence color images of the object (A) shown in FIGS. 6 to 9 are actually color images showing the colors of the fluorescence emissions generated from the surface of the object (A). The fluorescence images of the object (A) are images obtained by capturing images of a plurality of predetermined fluorescence emissions each having a different wavelength (450 nm, 500 nm, 550 nm, 600 nm, 650 nm, and 750 nm) extracted out of the fluorescence emissions generated from the surface of the object (A). The fluorescence images of the object (A) are obtained by using, for example, the determination device (1, 2).

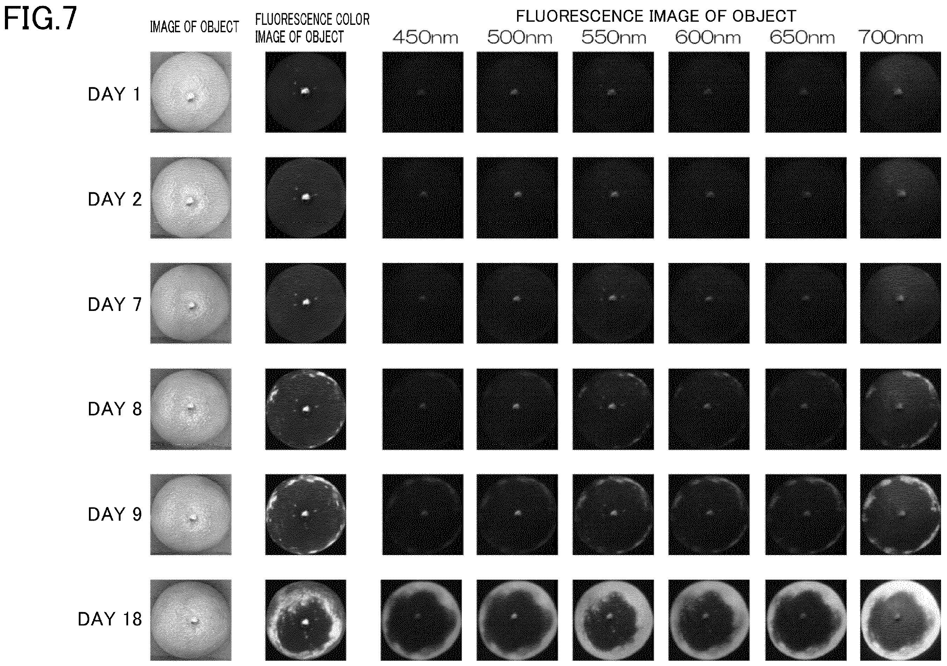

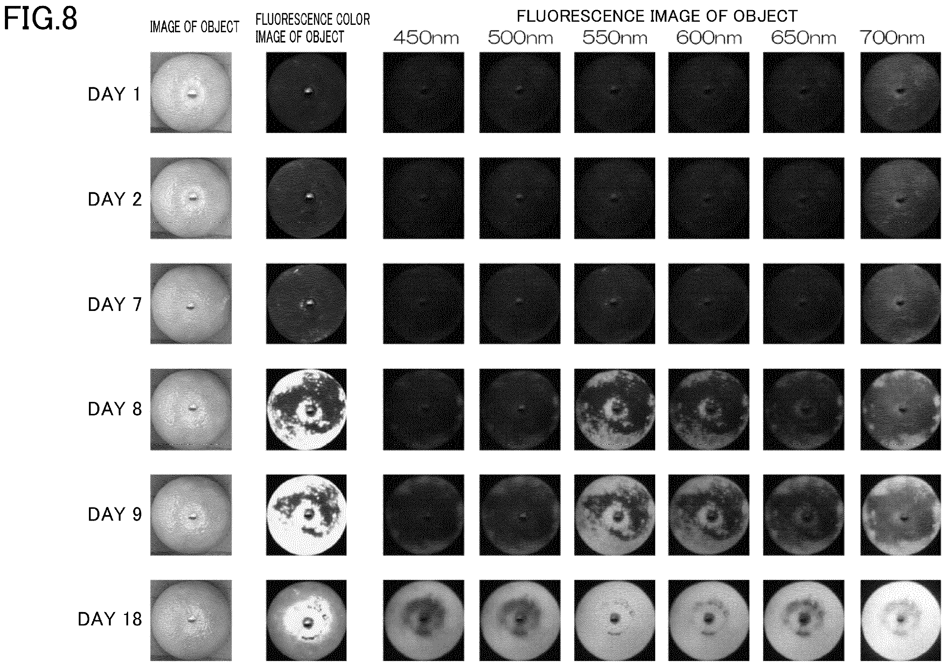

The data shown in FIG. 6 were obtained by observing a normal object (A). The data shown in FIG. 7 were obtained by observing the object (A) that was being disordered due to rot, a lesion, or the like caused by mold or the like with the passage of time. When the data shown in FIGS. 6 and 7 were acquired, the object (A) was placed in an environment of about 6°C, for example. The data shown in FIG. 8 were obtained by observing the object (A) that was refrigerated (e.g., at a temperature of about -1°C) and disordered by refrigeration with the passage of time. The disorder means that the object (A) is discolored in appearance.

The normal object (A) used to acquire the data shown in FIG. 6 may be hereinafter referred to as the "normal fruit." The object (A) that was being disordered due to rot, a lesion, or the like and that was used to acquire the data shown in FIG. 7 may be hereinafter referred to as a "first disordered fruit." The object (A) that was being disordered by refrigeration that was used to acquire the data shown in FIG. 8 may be hereinafter referred to as a "second disordered fruit."

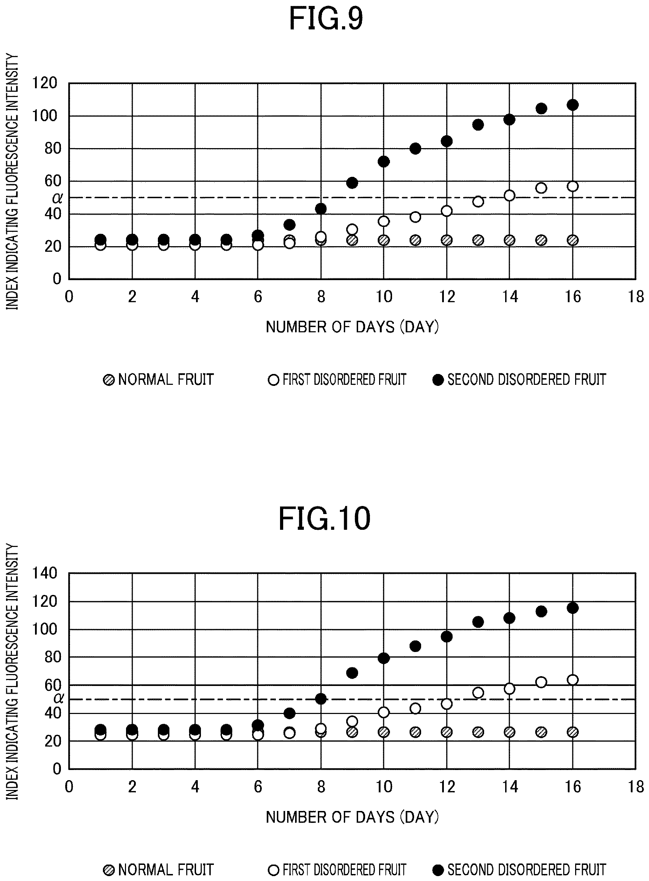

In FIGS. 9 to 14, the hatched circles indicate data on the normal fruit, the while hollow circles indicate data on the first disordered fruit, and the black-filled circles indicate data on the second disordered fruit. In the data shown in FIGS. 9 to 14, the vertical axis represents the index indicating the fluorescence intensity, and the horizontal axis represents the days of observation. The index indicating the fluorescence intensity is a value output based on pixel values of a plurality of pixels constituting the fluorescence image as described above. The greater the index indicating the fluorescence intensity is, the more the object (A) is disordered.

The data shown in FIG. 9 are created based on the data on the 450-nm fluorescence images among the data shown in FIGS. 6 to 8. FIG. 9 shows data indicating association between the observation days and the index indicating the fluorescence intensity of the associated 450-nm fluorescence image of each of the normal fruit, the first disordered fruit, and the second disordered fruit. For example, the index indicating the fluorescence intensity associated with the first disordered fruit (while hollow circles) on day 8 in the data shown in FIG. 9 is output based on pixel values of a plurality of pixels constituting the fluorescence image on day 8 in the data shown in FIG. 7.

FIG. 10 is created based on the data on the 500-nm fluorescence images among the data shown in FIGS. 6 to 8. FIG. 10 shows data indicating association between the observation days and the index indicating the fluorescence intensity of the associated 500-nm fluorescence image of each of the normal fruit, the first disordered fruit, and the second disordered fruit.

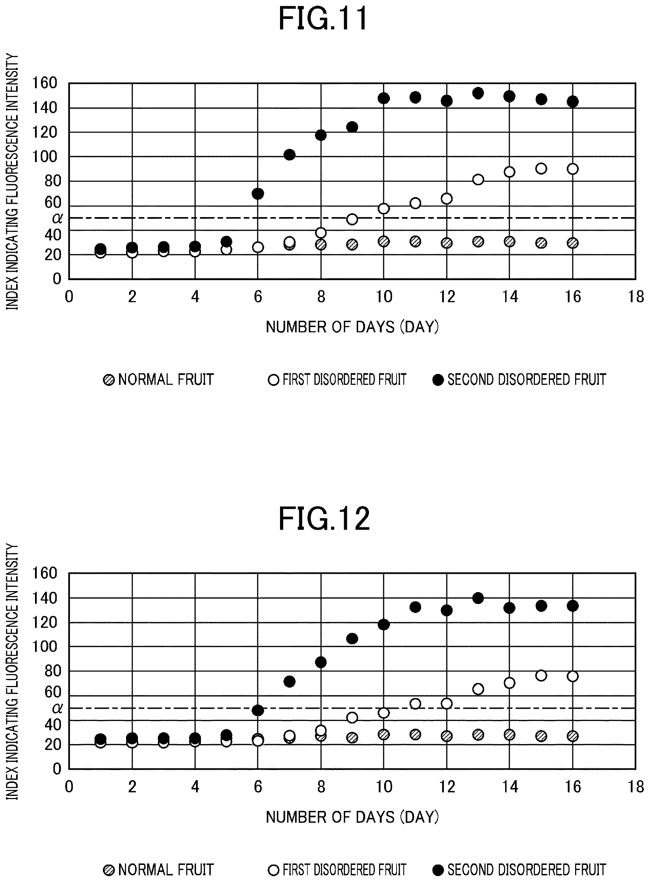

FIG. 11 is created based on the data on the 550-nm fluorescence images among the data shown in FIGS. 6 to 8. FIG. 11 shows data indicating association between the observation days and the index indicating the fluorescence intensity of the associated 550-nm fluorescence image of each of the normal fruit, the first disordered fruit, and the second disordered fruit.

FIG. 12 is created based on the data on the 600-nm fluorescence images among the data shown in FIGS. 6 to 8. FIG. 12 shows data indicating association between the observation days and the index indicating the fluorescence intensity of the associated 600-nm fluorescence image of each of the normal fruit, the first disordered fruit, and the second disordered fruit.

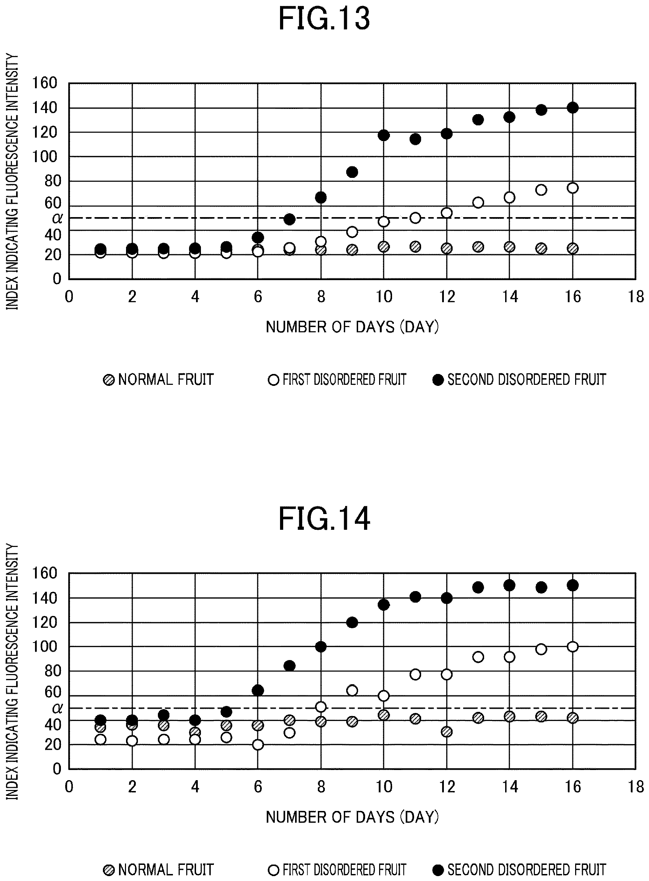

FIG. 13 is created based on the data on the 650-nm fluorescence images among the data shown in FIGS. 6 to 8. FIG. 13 shows data indicating association between the observation days and the index indicating the fluorescence intensity of the associated 650-nm fluorescence image of each of the normal fruit, the first disordered fruit, and the second disordered fruit.

FIG. 14 is created based on the data on the 700-nm fluorescence images among the data shown in FIGS. 6 to 8. FIG. 14 shows data indicating association between the observation days and the index indicating the fluorescence intensity of the associated 700-nm fluorescence image of each of the normal fruit, the first disordered fruit, and the second disordered fruit.

The inventors of this application confirmed that the normal fruit was not disordered and hardly discolored in appearance even if the number of the observation days increased, and thus the fluorescence images hardly varied, as illustrated in FIG. 6. The inventors of this application confirmed that the fluorescence images of the normal fruit hardly varied even if the number of the observation days increased, and thus the indexes indicating the fluorescence intensities of the fluorescence images also hardly varied, as illustrated in FIGS. 9 to 14.

The inventors of this application confirmed that each of the first and second disordered fruits was more disordered as the number of the observation days increased; thus each of the first and second disordered fruits was more discolored in appearance; and accordingly the change in the fluorescence image (the area of a whitish region) was larger, as illustrated in FIGS. 7 and 8. The inventors of this application confirmed each of the first and second disordered fruits was more disordered as the number of the observation days increased; the change in the fluorescence image (the area of the whitish region) was larger; and accordingly the index indicating the fluorescence intensity of the fluorescence image increased, as illustrated in FIGS. 9 to 14. In addition, the inventors of this application confirmed that the index indicating the fluorescence intensity of the fluorescence image of the second disordered fruit more increased than that of the first disordered fruit.

In an early phase of disorder of each of the first disordered fruit (see FIG. 7) and the second disordered fruit (see FIG. 8), i.e., in a phase (e.g., on day 9) where there was a sign of deterioration in quality thereof, a slight change in the color of the appearance of the object (A) was found when the image of the object (A) was visually checked. Thus, the inventors of the present application confirmed that it was difficult to visually check the image of the object (A) to determine whether or not there was a sign of deterioration in quality of the object (A).

In contrast, if there was a sign of deterioration in quality of each of the first disordered fruit (see FIG. 7) and the second disordered fruit (see FIG. 8), a whitish region appeared in the fluorescence image on day 9 in each of FIGS. 7 and 8, and thus there were increases in the index indicating the fluorescence intensity of the first disordered fruit (the while hollow circles) and the index indicating the fluorescence intensity of the second disordered fruit (the black-filled circles) on day 9 in FIGS. 9 to 14. Thus, the inventors of the present application confirmed that by checking the index indicating the fluorescence intensity of the fluorescence image, it could be determined whether or not there was a sign of deterioration in quality of the object (A).

The inventors of this application confirmed that if the object (A) was disordered (discolored) by sinensetin and nobiletin contained in the object (A), there appeared changes in the index indicating the fluorescence intensity of the 450-nm fluorescence image and the index indicating the fluorescence intensity of the 500-nm fluorescence image. Accordingly, the inventors of this application confirmed that the index indicating the fluorescence intensity of the 450-nm fluorescence image and the index indicating the fluorescence intensity of the 500-nm fluorescence image could be used as indexes for determining whether or not the object (A) was disordered (discolored) by sinensetin and nobiletin contained in the object (A). The inventors of this application confirmed that if the object (A) was disordered due to rot, there appeared a change in the index indicating the fluorescence intensity of the 550-nm fluorescence image. Accordingly, the inventors of this application confirmed that the index indicating the fluorescence intensity of the 550-nm fluorescence image could be used as an index for determining whether or not the object (A) was disordered by rot. The inventors of this application confirmed that the object (A) was disordered by Maillard reaction, there appeared changes in the index indicating the fluorescence intensity of the 600-nm fluorescence image and the index indicating the fluorescence intensity of the 650-nm fluorescence image. Accordingly, the inventors of this application confirmed that the index indicating the fluorescence intensity of the 600-nm fluorescence image and the index indicating the fluorescence intensity of the 650-nm fluorescence image could be used as indexes for determining whether or not the object (A) was disordered by Maillard reaction. The inventors of this application confirmed that the object (A) was disordered (discolored) by chlorophyll contained in the object (A), there appeared a change in the index indicating the fluorescence intensity of the 700-nm fluorescence image. Accordingly, the inventors of this application confirmed that the index indicating the fluorescence intensity of the 700-nm fluorescence image could be used as an index for determining whether or not the object (A) was disordered by chlorophyll contained in the object (A).

### -First Example of Operation of Determiner-

A first example of a procedure in which the determiner (60) of the determination device (1, 2) determines the sign of deterioration in quality of the object (A) will be described below.

The storage (50) (see FIGS. 1 and 4) of the determination device (1, 2) stores information indicating a predetermined threshold value α (see FIGS. 9 to 14). The determination device (1, 2) uses the threshold value α to determine whether or not the object (A) is disordered.

As illustrated in FIGS. 1, 4, and 15, in step S 10, the determiner (60) of the determination device (1, 2) performs a fluorescence image acquisition process. The fluorescence image acquisition process is a process in which the determiner (60) acquires data of a fluorescence image generated by the irradiator (10) irradiating the object (A) with light; the extractor (20) extracting a predetermined fluorescence emission generated from the surface of the object (A); and the imager (30) capturing the fluorescence image indicating the predetermined fluorescence emission. The determiner (60) acquires data of fluorescence images indicating a plurality of predetermined fluorescence emissions each having a different wavelength. In the tenth embodiment, the determiner (60) acquires a 450-nm fluorescence image (a fluorescence image indicating a predetermined fluorescence emission having a wavelength of 450 nm), a 500-nm fluorescence image, a 550-nm fluorescence image, a 600-nm fluorescence image, a 650-nm fluorescence image, and a 750-nm fluorescence image.

In step S20, the determiner (60) performs a fluorescence intensity output process. The fluorescence intensity output process is a process of outputting the index indicating the fluorescence intensity based on the pixel values of the plurality of pixels constituting each of the fluorescence images acquired in step S10. In the tenth embodiment, the determiner (60) outputs an index indicating the fluorescence intensity of each of the plurality of fluorescence images acquired in step S10. Specifically, the determiner (60) outputs the index indicating the fluorescence intensity of the 450-nm fluorescence image, the index indicating the fluorescence intensity of the 500-nm fluorescence image, the index indicating the fluorescence intensity of the 550-nm fluorescence image, the index indicating the fluorescence intensity of the 600-nm fluorescence image, the index indicating the fluorescence intensity of the 650-nm fluorescence image, and the index indicating the fluorescence intensity of the 750-nm fluorescence image.

In step S30, the determiner (60) determines whether or not at least one of the plurality of indexes each indicating the fluorescence intensity and output in step S20 is greater than the threshold value α. In the tenth embodiment, the determiner (60) determines whether or not at least one of the indexes is greater than the threshold value α among the index indicating the fluorescence intensity of the 450-nm fluorescence image, the index indicating the fluorescence intensity of the 500-nm fluorescence image, the index indicating the fluorescence intensity of the 550-nm fluorescence image, the index indicating the fluorescence intensity of the 600-nm fluorescence image, the index indicating the fluorescence intensity of the 650-nm fluorescence image, or the index indicating the fluorescence intensity of the 750-nm fluorescence image.

If the determiner (60) determines that at least one of the plurality of indexes each indicating the fluorescence intensity is greater than the threshold value α (Yes in step S30), the process proceeds to step S40.

If the determiner (60) determines that all of the plurality of indexes each indicating the fluorescence intensity are less than or equal to the threshold value α (No in step S30), the process proceeds to step S50.

In step S40, the determiner (60) determines that there is a sign of deterioration in quality of the object (A). As a result, the process ends.

In step S50, the determiner (60) determines that there is no sign of deterioration in quality of the object (A). If the determiner (60) determines that there is no sign of deterioration in quality of the object (A), the process proceeds to step S10, such that the processes shown in steps S10 to S30 are performed again. Then, the processes shown in steps S10 to S30 are repeated until the determiner (60) determines that there is a sign of deterioration in quality of the object (A). As a result, the processes shown in steps S10 to S30 are performed over time, and thus a temporal change in the object (A) can be observed.

As described above, the extractors (20) extract the plurality of predetermined fluorescence emissions each having a different wavelength; the imager (30) performs, over time, the imaging process of capturing a fluorescence image of each of the predetermined fluorescence emissions over time; and the determiner (60) determines whether or not there is a sign of deterioration in quality of the object (A) based on the indexes each indicating the fluorescence intensity of the associated fluorescence image acquired through the imaging process performed over time. As a result, by checking the indexes each indicating the fluorescence intensity of the associated fluorescence image acquired through the imaging process performed over time, it is possible to check a temporal change in the state of the object (A). In addition, by capturing a fluorescence image of each of the predetermined fluorescence emissions, it is possible to check a temporal change in the state of the object (A) in consideration of the causes (such as diseases, rot, and Maillard reaction) of disorders of the object (A) and of fluorescent materials (such as sinensetin, nobiletin, chlorophyll, heptamethoxyflavone, and carotin) deposited due to those causes.

### -Second Example of Operation of Determiner-

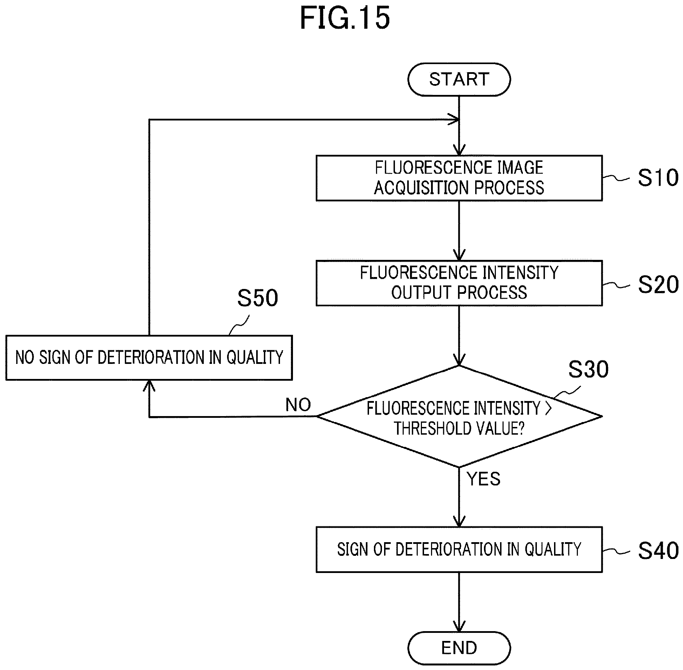

A second example of the procedure in which the determiner (60) of the determination device (1, 2) determines the sign of deterioration in quality of the object (A) will be described. Differences from the first example illustrated in FIG. 15 will be mainly described below.

As illustrated in FIGS. 1, 4, and 15, in step S10, the determiner (60) performs a fluorescence image acquisition process. In the second example, in the fluorescence image acquisition process, the determiner (60) acquires data of a fluorescence image indicating a predetermined fluorescence emission having one wavelength. For example, in the second example, in the fluorescence image acquisition process, the determiner (60) acquires data of a fluorescence image indicating a predetermined fluorescence emission having a wavelength of 500 nm.

In step S20, the determiner (60) performs a fluorescence intensity output process. In the second example, in the fluorescence intensity output process, the determiner (60) acquires an index indicating the fluorescence intensity of a fluorescence image indicating one predetermined fluorescence emission (e.g., a 500-nm fluorescence image).

In step S30, the determiner (60) determines whether or not the index indicating the fluorescence intensity and output in step S20 is greater than the threshold value α. If the determiner (60) determines that the index indicating the fluorescence intensity is greater than the threshold value α (Yes in step S30), the process proceeds to step S40. If the determiner (60) determines that the index indicating the fluorescence intensity is less than or equal to the threshold value α (No in step S30), the process proceeds to step S50.

As described above, the imager (30) captures a fluorescence image over time, and the determiner (60) determines whether or not there is a sign of deterioration in quality of the object (A) based on the index of the fluorescence image captured over time. As a result, in the fluorescence image acquisition process, the determiner (60) acquires data of a fluorescence image indicating a predetermined fluorescence emission having one wavelength, and thus the extractor (20) can have a more simple device configuration than in the first example in which data of the fluorescence image indicating the predetermined fluorescence emissions having a plurality of wavelengths are acquired.

### «Other Embodiments»

While the embodiments and variations thereof have been described above, it will be understood that various changes in form and details may be made without departing from the spirit and scope of the claims. The embodiments and the variations thereof may be combined and replaced with each other without deteriorating intended functions of the present disclosure.

### INDUSTRIAL APPLICABILITY

As can be seen in the foregoing description, the present disclosure is useful for a determination device.

### DESCRIPTION OF REFERENCE CHARACTERS

- 10: Irradiator
- 20: Extractor
- 30: Imager
- 60: Determiner
- 70: Scanning Mechanism
- 80: Switching Part
- A: Object

## Claims

1. A determination device comprising:
an irradiator (10) configured to irradiate an object (A) including a food or a plant with a light;
an extractor (20) configured to extract a predetermined fluorescence emission having a predetermined wavelength out of fluorescence emissions generated from a surface of the object (A) irradiated with the light;
an imager (30) configured to capture a fluorescence image indicating the predetermined fluorescence emission; and
a determiner (60) configured to determine a state of the object (A) based on an index indicating a fluorescence intensity of the fluorescence image.

2. The determination device of claim 1, wherein
the determiner (60) determines the state of the object (A) based on the fluorescence image including a plurality of fluorescence images, and
the predetermined fluorescence emission indicated by the fluorescence image has a different wavelength for each of the fluorescence images.

3. The determination device of claim 1 or 2, further comprising:
a scanning mechanism (70) configured to change relative positions of the object (A) and the imager (30) relative to each other.

4. The determination device of any one of claims 1 to 3, wherein
the predetermined wavelength is 550 nm or more and 750 nm or less.

5. The determination device of any one of claims 1 to 4, wherein
the irradiator (10) emits ultraviolet rays.

6. The determination device of claim 5, wherein
the ultraviolet rays have a wavelength of 200 nm or more and 400 nm or less.

7. The determination device of any one of claims 1 to 6, wherein
the imager (30) captures the fluoroscopic image over time, and
the determiner (60) determines whether or not there is a sign of deterioration in quality of the object (A) based on the index of the fluorescence image captured over time.

8. The determination device of any one of claims 1 to 6, wherein
the extractor (20) extracts the predetermined fluorescence emission including a plurality of predetermined fluorescence emissions each having a different wavelength,
the imager (30) performs an imaging process of capturing a fluorescence image of each of the predetermined fluorescence emissions over time, and
the determiner (60) determines whether or not there is a sign of deterioration in quality of the object (A) based on the index of each of the fluorescence images acquired through the imaging process performed over time.

9. The determination device of any one of claims 1 to 8, wherein
the extractor (20) includes a band-pass filter.

10. The determination device of claim 9, further comprising:
the band-pass filter including a plurality of band-pass filters; and
a switching part (80) configured to switch one of the plurality of band-pass filters that is used to extract the predetermined fluorescence emission,
wherein
each of the band-pass filters extracts the predetermined fluorescence emission having a different wavelength.

11. The determination device of any one of claims 1 to 10, further comprising:
the extractor (20) including a plurality of extractors (20); and
the imager (30) including a plurality of the imagers (30),
wherein
each of the extractors (20) extracts the predetermined fluorescence emission having a different wavelength, and
the plurality of extractors (20) are associated with the plurality of imagers (30).

12. The determination device of any one of claims 1 to 11, wherein
the irradiator (10) emits the light including a plurality of lights having different excitation wavelengths, and
the determiner (60) determines the state of the object (A) based on the index including a plurality of indexes associated with the plurality of lights.

13. The determination device of claim 12, wherein
the determiner (60) identifies a type of a live bacterium or a microorganism that is parasitic on the object (A) based on the plurality of indexes.
